# EUROPEAN PATENT APPLICATION

(11) **EP 4 102 516 A1**
(43) Date of publication of application: **14.12.2022**
(21) Application number: 21382524.3
(22) Date of filing: 11.06.2021
(51) Int. Cl.: G16H 50/20

(54) **COVID-19 SCREENING METHOD**

(71) Applicant: Institut d'Investigació Sanitària Pere Virgili, 43003 Tarragona (ES); Universitat Rovira I Virgili, 43003 Tarragona (ES)
(72) Inventor: AZELI JAROSCH, Youcef, 43204 Tarragona (ES); LLOBET VALERO, Eduard, 43007 Tarragona (ES); FERNÁNDEZ SABATER, Alberto, 43007 Tarragona (ES)
(74) Representative: Hoffmann Eitle

(57) **Abstract**

The present invention relates to a highly sensitive method for the screening of COVID-19 based on the use of selected symptoms and a simple olfactory test requiring the identification of a scent. It further relates to a data processing device and computer program related thereto.

## Description

### FIELD OF THE INVENTION

The present invention relates to the field of computer-implemented methods for the screening of infectious diseases. More specifically it relates to a highly sensitive method for the screening of COVID-19 based on the use of selected symptoms and a simple olfactory test requiring the identification of a scent. It further relates to a data processing device and computer program related thereto.

### BACKGROUND OF THE INVENTION

Since the first cases of severe acute respiratory syndrome coronavirus 2 (SARS-COV-2) were diagnosed in December 2019 in the Chinese city of Wuhan, coronavirus disease 2019 (COVID-19) has spread rapidly (1). The strategies put in place in the vast majority of countries to control the virus have been ineffective. On May 18, 2021, 162 million cases were diagnosed and 3.3 million deaths occurred worldwide (2). The first results about the safety and effectiveness of different types of vaccines have raised optimism in the scientific community due to the possibility of controlling COVID-19 (3). However, key elements that may affect the effectiveness of vaccines such as the time needed for their global introduction, the duration of vaccine immunity, or the consequences of the SARS-COV-2 variants especially in regions with fewer health resources are still unknown (4) (5). Recently the emergence of a new variant in India has brought the country's healthcare system to the edge of catastrophe (6).

The early detection of symptoms suggestive of infection, rapid and efficient testing, contact tracing and isolation are the basis of an effective screening strategy to control transmission of COVID-19 and decrease the disease burden on healthcare systems. The Achilles' heel in the fight against this disease is the large number of patients who are asymptomatic or have few symptoms like an ordinary cold and who are able to transmit the disease (7)(8)(9). The reference diagnostic tool for COVID-19 is reverse transcriptase polymerase chain reaction (RT-PCR). Its accessibility may be limited for low-resource healthcare systems and its cost and time requirements preclude its use as a mass triage tool. Recently a screening tool based on a machine learning model including clinical features and symptoms has been built to prioritize testing for COVID-19 (10). A predictive model for COVID-19 that included the combination of symptoms and wearable sensor data performed better than a model based on symptoms alone (11).

Olfactory dysfunction (OD) in COVID-19 patients has recently been described as one of the most prevalent symptoms (12) and could be used as screening to help identify people who could be recommended to self-isolate (13). A symptom predictive model for Covid-19 based on a smartphone app containing age, sex, perceived loss of smell and taste, persistent cough, severe fatigue and skipped meals obtained a sensitivity of 65% (14). At the time of diagnosis, a recent prospective study found that 31% of patients affected by COVID-19 presented OD (15). Between 11.8% and 23% of cases presented OD before any other symptoms (12) (16). A recent study, showed that a simplified clinical test based on the University of Pennsylvania Smell Identification Test was able to detect unperceived OD in COVID-19 patients (15).

Accordingly, there is a need for improved methods for the screening of COVID-19, providing an inexpensive, rapid and/or efficient detection of symptomatic and/or asymptomatic subjects infected with SARS-COV-2. In particular, there is a need to provide methods with high sensitivity for the detection of asymptomatic subjects infected with SARS-COV-2.

### SUMMARY OF THE INVENTION

The inventors have developed and validated using cross-validation techniques a predictive model for COVID-19 population identification using symptoms and a simple olfactory test based on an aromatized composition, such as an hydroalcoholic gel, which has shown high sensitivity for the detection of SARS-COV-2 infection. In the present invention, the terms SARS-COV-2 infection and COVID-19 are used indistinctly, unless otherwise specified. In particular, the capacity of this predictive model to detect infected SARS-COV-2 patients among asymptomatic subjects makes it a promising tool for the fight against COVID-19.

As shown at Table 6 different classification trees were built by machine learning according to the variables introduced in the model. The sensitive tree, privileges sensitivity over specificity, thus it minimizes false negative results being ideal for screening purposes where the objective is to rule out the disease. By considering the 8 relevant symptoms into the model, the sensitivity was 0.86 (95 Cl 0.79-0.92), the specificity was 0.37 (95% Cl 0.33-0.42) and the AUC was 0.86 (0.81-0.9) for the total population study. Sensitivity raised to 0.94 (95%CI 0.88-0.98) whilst specificity remained at 0.32 (95% Cl 0.28-0.37) for the total population study when the olfactory test was introduced into the model.

As shown in Table 7, it was further surprisingly found by the inventors that by selecting only 4 of the identified 8 relevant symptoms, namely fever, myalgia, diarrhoea and headache, in combination with the olfactory test, enabled to obtain values of sensitivity equally high. In particular with a sensitivity of 0.94 (95% Cl 0.88-0.98) was obtained with an AUC of 0.87 (95% Cl 0.83-0.92), although specificity decreased to 0.24 (95% Cl 0.20-0.28) for the total population study. The increase in sensitivity when adding the olfactory test to this combination of symptoms is even more significant than for the relevant symptoms described in Table 6, namely from 0.79 (95% Cl 0.70-0.86) for the 4 symptoms to 0.94 (95% Cl 0.88-0.98) when combined with the olfactory test.

The possible applications of the predictive model and related methods and device described herein are diverse. In a situation of community transmission, the early detection of subjects infected with SARS-COV-2 is the basis of case control and burden reduction of the COVID-19 on the healthcare system. In situations where the incidence of the disease is high, mass testing strategies using diagnostic tests such as antigen tests or PCR are used, but difficult to organize and have a high cost. Therefore, massive screening based on these methods may not be maintained over long timeframes. The predictive model, methods and device described herein could be useful for the screening of the general population and selecting those individuals who could benefit from a more expensive diagnostic test such as antigen testing or RT-PCR.

Moreover, because of its simplicity (it is easy to perform and does not require a specialist operator or setting), portability and low manufacturing costs, the method based on this predictive model could be especially useful for population screening, e.g., as a point-of-care test, when testing resources are limited. Moreover, it is a rapid test which results can be provided within minutes.

The first aspect of the invention relates to a computer implemented (CI) method for the screening of subjects infected with SARS-COV-2 virus, wherein said method comprises:
i. collecting information from a subject on the perceived presence of clinical symptoms comprising or consisting of one or more selected from the group consisting of fever, cough (dry cough or productive cough), myalgia, headache, diarrhoea, asthenia, perceived altered sense of smell (OD), and perceived altered sense of taste (GD);
ii. collecting the results of an olfactory test requiring the identification of a scent by the subject, preferably wherein said olfactory test consists of a single testing step, wherein when the scent is not identified, the olfactory test is annotated as positive;
iii. processing the information obtained from steps i) and ii) by a computer to generate an output information identifying or classifying a subject having or with a high likelihood to have SARS-COV-2 infection.

The second aspect of the invention relates to a device for the screening of subjects infected with SARS-COV-2 virus, comprising:
one or more means for collecting the information regarding the clinical condition of the subject and the results of an olfactory test as described herein, and
means for generating information to generate and output information,
wherein the means for generating information is constituted so that
   A) during a first collection step, information relating to clinical conditions of the subject is collected, comprising the perceived presence of clinical symptoms comprising or consisting of one or more selected from the group consisting of fever, cough (dry cough or productive cough), myalgia, headache, diarrhoea, asthenia, perceived altered sense of smell (OD), and perceived altered sense of taste (GD);
   B) during a second collection step the results of an olfactory test requiring the identification of a scent by the subject are collected, wherein when the scent is not identified, the olfactory test is annotated as positive; and
   C) processing the information collected in A) and B) and generating an output information identifying said subject as having or with a high likelihood to have SARS-COV-2 infection.

In a third aspect the present invention relates to a computer program capable of implementing step iii) of the methods described herein or for generating the output information in C) of the device described herein.

### BRIEF DESCRIPTION OF THE DRAWINGS

**Figure 1****:** This graph illustrates the D-limonene's concentration in the volatile fraction for different concentrations of the lemon essential oil (Limón España^{®}) in the hydroalcoholic gel composition by GC-MS analysis.
**Figure 2****:** This graph illustrates the (i) accumulated number of cases included in the study, (ii) the RT-PCRs performed per week in the study health centres; and (iii) 14-days cumulative number of SARS-Cov-2 infections per 100.000 inhabitants in the city of Reus during the study period.
**Figure 3****:** Flow Chart summarizing the results of the prospective study. The reference test (gold standard) was RT-PCR test for the detection of SARS-COV-2.
**Figure 4****:** ROC curve of the sensitive classification tree algorithm using relevant symptoms and olfactory test in the general population.
**Figure 5****:** Decision tree including relevant symptoms + olfactory test + sex + age.

### DETAILED DESCRIPTION OF THE INVENTION

### Definitions

The terms "subject", or "individual"' are used herein interchangeably to refer to all the animals classified as mammals and includes but is not limited to domestic and farm animals, primates and humans, for example, human beings, non-human primates, cows, horses, pigs, sheep, goats, dogs, cats, or rodents. Preferably, the subject is a male or female human being of any age or race.

The term "screening" is understood herein as the examination or testing of a group of individuals with the objective of discriminating healthy individuals from those who have or are suspected of having a disease. A method of screening is generally used for the "early detection" of a disease. The expression "early detection" refers to detection before the presence of clinical signs.

The term "diagnosis", as used herein, refers both to the process of attempting to determine and/or identify a possible disease in a subject, i.e. the diagnostic procedure, and to the opinion reached by this process, i.e. the diagnostic opinion.

Sensitivity, specificity, and/or accuracy are parameters typically used to describe the validity or performance of a test. In particular, they are used to quantify how good and reliable the discrimination method is. A test is usually calibrated in terms of the desired specificity and sensitivity according to the target use of the test in clinical practice. High sensitivity corresponds to high negative predictive value and is considered generally a desired property for a "rule out" test, such as a screening test which typically will be followed by a confirmatory test. High specificity corresponds to high positive predictive value and is considered generally a desired property for a "rule in" test, such as a companion diagnostic test.

The term "sensitivity" as used herein refers to the proportion of subjects who have the target condition (reference standard positive) and give positive test results (TP/ (TP + FN)). It shows how good the test is at detecting a disease. Sensitivity ("sens") may be within the range of 0 (0%) < sens < 1 (100%) and ideally, the number of false negatives equaling zero or close to equaling zero and sensitivity equaling one (100%) or close to equaling one (100%).

The term "specificity" as used herein in connection with a test refers to the proportion of subjects without the target condition (reference standard negative) and give negative test results (TN/ (TN + FP)). It shows how good the test is at identifying normal (negative) condition. Specificity ("spec") may be within the range of 0 (0%) < spec < 1 (100%) and ideally, the number of false positives equaling zero or close to equaling zero and specificity equaling one (100%) or close to equaling one (100%).

The term "accuracy" as used herein refers to the proportion of true results, either true positive or true negative, in a population. It measures the degree of veracity of a screening test on a condition, i.e., how correct is the determination and exclusion of a given condition (TN + TP)/(TN+TP+FN+FP). Accuracy ("acc") may be within the range of 0 (0%) < acc < 1 (100%) and ideally, the number of false positives and false negative equaling zero or close to equaling zero and accuracy equaling one (100%) or close to equaling one (100%).

The term "Receiver Operating Characteristic (ROC) curves" as used herein refers to a graphical plot that illustrates the performance of a binary classifier system as its discrimination threshold is varied. The curve is created by plotting the true positive rate against the false positive rate at various threshold settings. The true positive rate is also known as sensitivity. The false positive rate is calculated as 1 - specificity. The ROC curve is thus a way of graphically displaying the true positive rate versus the false positive rate (sensitivity vs (1-specificity)) across a range of cut-offs and of selecting the optimal cut-off for clinical use. Accuracy expressed as the area under the ROC curve (AUC) provides a useful parameter for comparing test performance. An AUC approaching 1 indicates that the test is highly sensitive as well as highly specific whereas an AUC approaching 0.5 indicates that the test is neither sensitive nor specific. In general, a test is considered to be a suitable discriminator if the AUC is from 0.6 to 0.75, to have good discrimination capacity if the AUC is from 0.75 to 0.9 and to be an excellent discriminator if the AUC is from 0.9 to 1. For further details see for instance, Zweig MH and Campbell G, Clinical Chemistry 1993; 39:561-577 or Greiner et al. Preventive Veterinary Medicine 2000; 45:23-41.

### Detailed description

In a first aspect, the present invention relates to a computer implemented (CI) method for the screening of subjects infected with SARS-COV-2 virus, wherein said method comprises:
i. collecting information from a subject on the perceived presence of clinical symptoms;
ii. collecting the results of an olfactory test requiring the identification of a scent by the subject, wherein when the scent is not identified, the olfactory test is annotated as positive;
iii. processing the information obtained from steps i) and ii) by a computer to generate an output information identifying or classifying a subject as having or with a high likelihood to have SARS-COV-2 infection.

The subjects may be from a random sample of the general population or pre-selected for having characterizing features, such as sex, age, one or more COVID-19 symptoms or other risk factors. Said subjects are preferably from a random sample of the general population and includes symptomatic and asymptomatic individuals and may or not have one or more risk factors (e.g., having been in contact with a COVID-19 positive subject). Symptomatic subjects may be those having one or more of the symptoms in Table 3. In some embodiments in point i) information may also be collected regarding the presence of risk factors such as being a close contact of a COVID-19 positive subject. Close contacts may be considered those persons who had shared space with a positive case at a distance of less than 2 meters, for more than 15 minutes, without protection and from 48 hours prior to the onset of symptoms.

As shown in Table 3, the inventors found 8 from the 14 symptoms tested to be statistically significant for SARS-COV-2 infection in a logistic regression predictive model and these are referred herein as "relevant symptoms". With the aim to increase the applicability of the predictive method of the invention, the productive cough variable was also included as a relevant symptom. Therefore, no distinction was made between dry and productive cough, and "cough" was used as a variable of the "relevant symptoms" in the results shown in Table 6.

Said clinical symptoms in step i) comprise or consist of one or more selected from the group consisting of fever, cough (dry cough or productive cough), myalgia, headache, diarrhoea, asthenia, perceived altered sense of smell (OD), and perceived altered sense of taste (GD).

In particular embodiments, said clinical symptoms in step i) comprise or consist of combinations of at least two, three, four, five, six, seven or eight of these symptoms. Illustrative combinations of the relevant symptoms include the following:

### Two symptoms' combinations

fever + cough (dry cough or productive cough), fever + myalgia, fever + headache, fever + diarrhoea, fever + asthenia, fever + OD or fever + GD;
cough (dry cough or productive cough)+ myalgia, cough (dry cough or productive cough)+ headache, cough (dry cough or productive cough)+ diarrhoea, cough (dry cough or productive cough)+ asthenia, cough (dry cough or productive cough)+ OD or cough (dry cough or productive cough)+ GD;
myalgia + headache; myalgia + diarrhoea; myalgia + asthenia; myalgia + OD or + myalgia + GD;
headache + diarrhoea, headache + asthenia, headache + OD or headache + GD;
diarrhoea + asthenia, diarrhoea + OD or diarrhoea + GD;
asthenia + OD, asthenia + GD or OD + GD.

### Three symptoms' combinations

fever + cough (dry cough or productive cough)+ myalgia, fever + cough (dry cough or productive cough)+ headache, fever + cough (dry cough or productive cough)+ diarrhoea, fever + cough (dry cough or productive cough)+ asthenia, fever + cough (dry cough or productive cough)+ OD or fever + cough (dry cough or productive cough) + GD;
fever + myalgia + headache, fever + myalgia + diarrhoea, fever + myalgia + asthenia, fever + myalgia + OD or fever + myalgia + GD;
fever + headache + diarrhoea, fever + headache + asthenia, fever + headache + OD or fever + headache + GD;
fever + diarrhoea + asthenia, fever + diarrhoea + OD or fever + diarrhoea + GD;
cough (dry cough or productive cough)+ myalgia + headache, dry cough + myalgia + diarrhoea, dry cough + myalgia + asthenia, cough (dry cough or productive cough)+ myalgia + OD or cough (dry cough or productive cough)+ myalgia + GD;
myalgia + headache + diarrhoea, myalgia + headache + asthenia, myalgia + headache + OD or myalgia + headache + GD;
headache + diarrhoea + asthenia; headache + diarrhoea + OD or headache + diarrhoea + GD; diarrhoea + asthenia + OD or diarrhoea + asthenia + GD.

### Four symptoms' combinations

fever + cough (dry cough or productive cough)+ myalgia + headache;
fever + cough (dry cough or productive cough)+ myalgia + diarrhoea;
fever + cough (dry cough or productive cough)+ myalgia + asthenia;
fever + cough (dry cough or productive cough)+ myalgia + OD;
fever + cough (dry cough or productive cough)+ myalgia + GD;
fever + cough (dry cough or productive cough)+ headache + diarrhoea;
fever + cough (dry cough or productive cough)+ headache + asthenia;
fever + cough (dry cough or productive cough)+ headache + OD;
fever + cough (dry cough or productive cough)+ headache + GD;
fever + cough (dry cough or productive cough)+ diarrhoea + asthenia;
fever + cough (dry cough or productive cough)+ diarrhoea + OD;
fever + cough (dry cough or productive cough)+ diarrhoea + GD;
fever + cough (dry cough or productive cough)+ asthenia + OD;
fever + cough (dry cough or productive cough)+ asthenia + GD;
fever + myalgia + headache + diarrhoea;
fever + myalgia + headache + asthenia;
fever + myalgia + headache + OD;
fever + myalgia + headache + GD;
fever + headache + diarrhoea + asthenia;
fever + headache + diarrhoea + OD;
fever + headache + diarrhoea + GD;
fever + diarrhoea + OD + GD;
cough (dry cough or productive cough)+ myalgia + headache + diarrhoea;
cough (dry cough or productive cough)+ myalgia + headache + asthenia;
cough (dry cough or productive cough)+ myalgia + headache + OD;
cough (dry cough or productive cough)+ myalgia + headache + GD;
cough (dry cough or productive cough)+ headache + diarrhoea + asthenia;
cough (dry cough or productive cough)+ headache + diarrhoea + OD;
cough (dry cough or productive cough)+ headache + diarrhoea + GD;
cough (dry cough or productive cough)+ diarrhoea + OD + GD;
myalgia + headache + diarrhoea + asthenia
myalgia + headache + diarrhoea + OD;
myalgia + headache + diarrhoea + GD;
headache + diarrhoea + asthenia + OD;
headache + diarrhoea + asthenia + GD.

In a preferred embodiment, step i) comprises collecting information from a subject on the perceived presence of clinical symptoms comprising or consisting of fever, myalgia, diarrhoea, headache, cough (dry cough or productive cough), asthenia, OD and GD. In another preferred embodiment, said clinical symptoms comprise or consist of fever, myalgia, diarrhoea and headache. In still another embodiment, said clinical symptoms comprise or consist of fever, myalgia, diarrhoea, headache and cough (dry cough or productive cough).

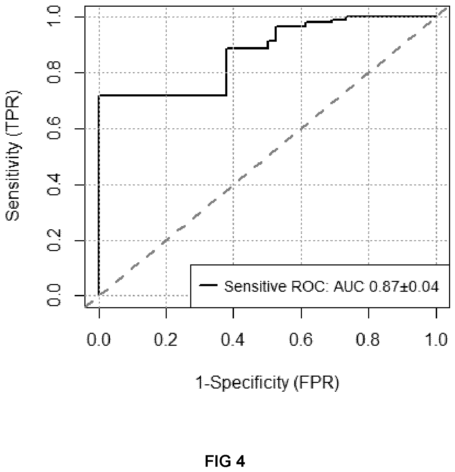

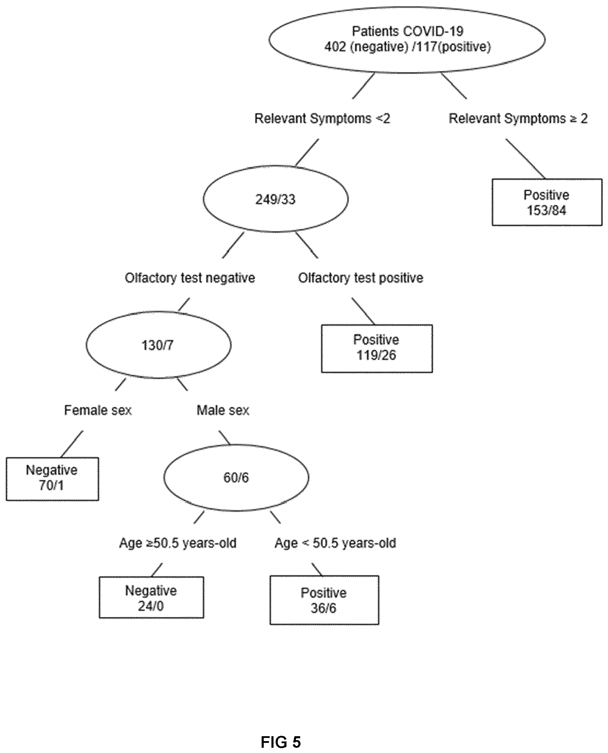

As shown in Table 6and Figure 4, the addition of the olfactory test to the relevant symptoms increased sensitivity up to 0.94 (95%CI 0.88-0.98) with an AUC of 0.87 (0.83-0.92) whilst specificity remained at 0.32 (95% Cl 0.28-0.37) for the total population study. The decision tree leading to these sensitivity, specificity and AUC values is shown in **Figure 5****.** Accordingly, in a more preferred embodiment, the method of the invention comprises:
i. collecting information from a subject on the perceived presence of clinical symptoms comprising or consisting of fever, myalgia, diarrhoea, headache, cough, asthenia, OD and GD;
ii. collecting the results of an olfactory test requiring the identification of a scent by the subject, preferably wherein said olfactory test consists of a single testing step, wherein when the scent is not identified, the olfactory test is annotated as positive; and
iii. generating an output information identifying said subject as having or with a high likelihood to have SARS-COV-2 infection when
   said subject has two or more of the clinical symptoms recited in step i); or
   when said subject has zero or one of the clinical symptoms recited in step i) and has a positive olfactory test in ii).

In a preferred embodiment, an output information is generated in step iii) identifying said subject as having or with a high likelihood to have SARS-COV-2 infection when said subject has in step i) fever, myalgia, diarrhoea and headache; and has a positive olfactory test in ii).

As shown in Table 7, the combination of fever, myalgia, diarrhoea and headache with an olfactory test as described herein provided values of sensitivity of 0.94 (95% Cl 0.88-0.98), AUC of 0.87 (95% Cl 0.83-0.92), with a specificity of 0.24 (95% Cl 0.20-0.28) for the total population study.

In a further embodiment, optionally in combination with any of the above, step i) further comprises collecting information from the subject's age and/or sex, preferably sex and age. The variable sex provides a binary classification: male or woman. With regard to age, this variable can be used to classify the subject according to an age threshold or range, for instance to stratify the subjects depending on whether or not they reach a certain age threshold, such as more than 40 years, more than 45 years, more than 50 years, more than 55 years, etc. As shown in Tables 5 and 6, the highest specificities were obtained when age and/or sex were further added to the predictive model.

In still a preferred embodiment, optionally in combination with any of the above, the method of the invention comprises:
i. collecting information from a subject on the perceived presence of clinical symptoms comprising or consisting of fever, myalgia, diarrhoea, headache, cough, asthenia, OD and GD and further collecting information on the subject's age and sex;
ii. collecting the results of an olfactory test requiring the identification of a scent by the subject, preferably wherein said olfactory test consists of a single testing step, wherein when the scent is not identified, the olfactory test is annotated as positive; and
iii. generating an output information identifying said subject as having or with a high likelihood to have SARS-COV-2 infection when
said subject has two or more of the clinical symptoms recited in step i); or
when said subject has zero or one of the clinical symptoms recited in step i) and has a positive olfactory test in ii); or
when said subject has zero or one of the clinical symptoms recited in step i) and has a negative olfactory test in ii), is a man and has 50 or less years.

The olfactory test used in the method of the invention comprises the identification of a scent or aroma, wherein the subject has to correctly identify the type of scent. The subject may be given a series of options from which to choose or simply asked to identify the scent. The second option being preferred. For instance, when being confronted to a citric scent (e.g. lemon) as described herein the subject has to identify it as a citric scent. It will be considered a negative result as far as it is identified as citric or any subcategory therein such as as lemon, citrus, orange, tangerine, citronella, or lime. Otherwise, the result will be considered positive.

Said olfactory test may comprise one or more steps. In a particular embodiment, said olfactory test consists of a single testing step, in other words wherein the subject is exposed to a scent and requested to identify it a single time. In another embodiment, said olfactory test comprises two steps, wherein the first step comprises testing for recognition of a scent in a first aromatized composition and the second step comprises testing for recognition of said or another scent in a second aromatized composition. The subject may be exposed to the test scent in different forms. Some illustrative non-limiting embodiments are provided herein. In some embodiments, the aromatized composition can be in the form of a solution, suspension or emulsion which is sprayed in front of the subject who is asked to identify the scent. In other embodiments, the aromatized composition is found in a recipient and the subject is asked to approach it to his/her nose and identify the scent. In still other embodiments, the subject may be asked to rub the composition in his/her skin (e.g. in the arm or hands) and identify the scent.

In some embodiments, said first and second tests require the recognition of different scents or aromas. In other embodiments, said first and second tests require the recognition of the same scent (e.g. a citric scent) but in different concentrations. For instance, it may require first the detection of a scent at a lower concentration of the aromatizing compound in a test composition and if it is not detected then the second test is conducted using a higher concentration of the aromatizing compound.

Said aromatizing compound may be present at a concentration from 0.01% w/w or v/w to 10% w/w or v/w, preferably at a concentration from 0.1% w/w or v/w to 1% w/w or v/w, more preferably at a concentration from 0.3% w/w or v/w to 0.5% w/w or v/w. In a preferred embodiment, said olfactory test comprises a single test wherein the aromatizing compound is found in the test composition at a concentration from 0.3% w/w or v/w to 0.5% w/w or v/w, preferably about 0.3% w/w or v/w. In another preferred embodiment, said test comprises two steps, wherein the aromatizing compound is found in the first test composition at a concentration of about 0.3% w/w or v/w and in the second test composition at a concentration of about 0.5% w/w or v/w.

The aromatizing compound can be any volatile aromatic compound. For instance, said scent can be that of an essential oil comprised in an aromatized, preferably otherwise odour-neutral composition, such as a solution, suspension, emulsion, cream, ointment, gel, such as an hydroalcoholic gel, or other well-known compositions. In particular embodiments, said composition comprises well known pharmaceutical excipients and/or carriers, in particular for topic formulations. Pharmaceutical excipients and carriers are nontoxic to recipients at the dosages and concentrations employed and are well known in the art, see for instance those discussed in Remington's Pharmaceutical Science (17th Ed., Mack Publishing Co., Easton, Pa., 1985) and Goodman and Gilman's The Pharmaceutical Basis of Therapeutics (8th Ed., Pergamon Press, Elmsford, N.Y., 1990) both of which are incorporated herein by reference. Hydroalcoholic gels are widely distributed being one of the main strategies to decrease virus transmission (17). These contain an alcohol (e.g. ethanol), water and may further contain a thickening agent, a preservative, a pH buffering agent or other. Thickening agents which may be used in the hydroalcoholic gel composition include but are not limited to glycerine, polyacrylic acid and polyethylene glycol, preferably the thickening agent is polyacrylic acid (e.g. Carbopol ^{®}).

Fragrance essential oils such as lavender, eucalyptus or lemon make the hydroalcoholic gels more pleasant and can enhance their anti-viral effect (18)(19). As shown by the inventors, the essential oils' features make a composition as described herein, such as an aromatized hydroalcoholic gel, a good candidate as a simple, fast and cost-effectiveness olfactory large scale screening test. Different flavors may be used depending on the local culture to facilitate the acceptance of the predictive tool. In a particularly preferred embodiment, said essential oil is a citric fruit essential oil, such as an sweet or bitter orange, tangerine, lemon, citronella, or lime essential oil. Typically, citric fruit essential oils are characterized by comprising D-limonene as one of the main volatile components in their composition. In a more preferred embodiment, said essential oil is a lemon essential oil, such as Limón España used in the Examples.

In some embodiments, said test comprises two steps, wherein the aromatizing compound is a citric essential oil as described herein and is found in the first test composition at a concentration of about 0.3% v/w and in the second test composition at a concentration of about 0.5% v/w. In a preferred embodiment, said olfactory test comprises a single test wherein the aromatizing compound is a citric essential oil as described herein above found in the test composition at a concentration from 0.3% v/w to 0.5% v/w, preferably about 0.3% v/w. The inventors found that including a second testing step in the olfactory test did not increase the sensitivity of the method (see Table 5, Olfactory test results) and indeed the second test was left out from the different sensitive classification trees built by machine learning (Tables 6 and 7).

The composition used for the olfactory test described herein can be presented or packaged in a small format, such as using sachets for individual use or in a dispenser comprising the aromatized composition described herein above. Such format is easy to handle and transport and facilitates its use as a screening test, for instance, it can be used prior to granting access to a public building, including a cinema, theatre or any cultural or sports event or in the workplace.

In some embodiments, said method is for COVID-19 screening purposes and has sensitivity values of at least 90%, preferably of at least 91%, 92%, 93%,94%, 95%,96%, 97%, 98% or 99%.

In some embodiments, the subject identified or classified as having or with a high likelihood to have SARS-COV-2 infection by a method of the invention as described herein is further submitted to a confirmation diagnostic test. The gold standard is SARS-COV-2 RT- PCR, for instance by using VIASURE SARS-COV-2 Real Time PCR Detection Kit (CerTest Biotec, Zaragoza, Spain), or Procleix1 method in a Panther automated extractor and amplifier (Grifols Laboratories, Barcelona, Spain) as described in the Examples. Antigen and molecular tests for diagnosing and ruling out COVID-19 are known in the art. These are pinpointed for instance at the Summary of findings 1 table at Dinnes J. et al. 2021 (30).

The collection of the results in steps i) and ii) can be conducted by requesting the subject to respond to a questionnaire, such as an on-line questionnaire. Such on-line questionnaire can be accessed from a terminal device by any means, such as by scanning a QR or BIDI code which can be displayed in the surface of the materials packaging the composition for the olfactory test or in another surface next to or attached to it. Alternatively, the questionnaire can be displayed for instance in a screen comprised in a device as discussed under the second aspect of the invention and the subject, or a third party on his behalf such as a medical practitioner, can introduce the information in the device, for instance by interacting with the screen displaying the questionnaire.

In a second aspect, the invention refers to a device for the screening of subjects infected with SARS-COV-2 virus as described herein, comprising:
one or more means for collecting the information regarding the clinical conditions of the subject and the results of an olfactory test as described herein, and
means for generating information to generate and output information,
wherein the means for generating information is constituted so that
   A) during a first collection step, information relating to clinical conditions of the subject as described herein above, is collected;
   B) during a second collection step the results of an olfactory test requiring the identification of a scent by the subject are collected, wherein when the scent is not identified, the olfactory test is annotated as positive; and
   C) an output information is generated based on the information collected in steps A) and B) identifying a subject having or with a high likelihood to have SARS-COV-2 infection.

Particular embodiments relating to the clinical conditions in step A) and the olfactory test in step B) are as defined herein above for the method of the invention.

In some embodiments, in step A) said clinical symptoms comprise or consist of fever, myalgia, diarrhoea, headache, cough, asthenia, OD, and GD; and
when said subject has two or more of the clinical symptoms recited in step A); or
when said subject has zero or one of the clinical symptoms recited in step A) and a positive olfactory test in step B) an output information is generated in step C) identifying said subject as having or with a high likelihood to have SARS-COV-2 infection.

In some embodiments, when there is presence of at least fever, myalgia, diarrhoea and headache from the clinical symptoms recited in step A), and a positive olfactory test in step B) an output information is generated in step C) identifying said subject as having or with a high likelihood to have SARS-COV-2 infection.

In some embodiments, optionally in combination with any of the above, in step B) said olfactory test comprises a single testing step as defined herein above.

The means for generating an output information can be a processing unit, which can be constituted with a CPU inside a computer and appropriate program, for classifying the subject based on the collected information. The output information can be for instance in the form of alert signals or word text, such as "positive" or "recommendation to conduct a confirmation test" appearing in a display.

The medical professional observing the generated output information indicating that the subject is identified or classified as presenting SARS-COV-2 infection may take the necessary medical procedures such as performing a diagnostic test, or referring the subject so that a diagnostic test is performed, to confirm the results. In other embodiments, when the subject sees the generated output information may respond by going to or contacting a medical institution by himself and requesting a confirmatory test to be conducted, such as antigen test or RT-PCR.

It is further noted that, in some embodiments the device of the second aspect of the invention, may further comprise, a data storage system to store the operating protocol and the questionnaire to collect the information on clinical conditions and the olfactory test, and a terminal display for the subject to visualize the questions and the generated output information. Such display can be replaced by any other manner of presenting the clinical questionnaire to the subject or medical professional, such as any suitable interacting means with them. The device may in addition provide means, preferably input means, that credibly assists the subject or third party, such as medical personnel, in completing the questionnaire by means of a continued and/or guided human-machine interaction process. Therefore, the device should further comprise, preferably a data storage system, a display for the subject to visualize the questionnaire and output information and means that assists the subject in responding to the questionnaire by means of a continued and/or guided human-machine interaction process.

In a further embodiment, the present invention relates to a computer program capable of implementing step iii) of the methods described herein or for generating the output information in C) of the device described herein.

Furthermore, the present disclosure further refers to an aromatized composition as described herein for use in the olfactory screening test of the method for the screening for COVID-19 described herein.

It is contemplated that any features described herein can optionally be combined with any of the embodiments of any method, device and computer program of the invention; and any embodiment discussed in this specification can be implemented with respect to any of these. It will be understood that particular embodiments described herein are shown by way of illustration and not as limitations of the invention.

All publications and patent applications are herein incorporated by reference to the same extent as if each individual publication or patent application was specifically and individually indicated to be incorporated by reference.

The use of the word "a" or "an" may mean "one," but it is also consistent with the meaning of "one or more," "at least one," and "one or more than one". The use of the term "another" may also refer to one or more. The use of the term "or" in the claims is used to mean "and/or" unless explicitly indicated to refer to alternatives only or the alternatives are mutually exclusive.

As used in this specification and claim(s), the words "comprising" (and any form of comprising, such as "comprise" and "comprises"), "having" (and any form of having, such as "have" and "has"), "including" (and any form of including, such as "includes" and "include") or "containing" (and any form of containing, such as "contains" and "contain") are inclusive or open-ended and do not exclude additional, unrecited elements or method steps. The term "comprises" also encompasses and expressly discloses the terms "consists of" and "consists essentially of". As used herein, the phrase "consisting essentially of" limits the scope of a claim to the specified materials or steps and those that do not materially affect the basic and novel characteristic(s) of the claimed invention. As used herein, the phrase "consisting of" excludes any element, step, or ingredient not specified in the claim except for, e.g., impurities ordinarily associated with the element or limitation.

The term "or combinations thereof" as used herein refers to all permutations and combinations of the listed items preceding the term. For example, "A, B, C, or combinations thereof" is intended to include at least one of: A, B, C, AB, AC, BC, or ABC, and if order is important in a particular context, also BA, CA, CB, CBA, BCA, ACB, BAC, or CAB. Continuing with this example, expressly included are combinations that contain repeats of one or more item or term, such as BB, AAA, AB, BBC, AAABCCCC, CBBAAA, CABABB, and so forth. The skilled artisan will understand that typically there is no limit on the number of items or terms in any combination, unless otherwise apparent from the context.

As used herein, words of approximation such as, without limitation, "about", "around", "approximately" refers to a condition that when so modified is understood to not necessarily be absolute or perfect but would be considered close enough to those of ordinary skill in the art to warrant designating the condition as being present. The extent to which the description may vary will depend on how great a change can be instituted and still have one of ordinary skilled in the art recognize the modified feature as still having the required characteristics and capabilities of the unmodified feature. In general, but subject to the preceding discussion, a numerical value herein that is modified by a word of approximation such as "about" may vary from the stated value by ±1, 2, 3, 4, 5, 6, 7, 8, 9, or 10%. Accordingly, the term "about" may mean the indicated value ± 5% of its value, preferably the indicated value ± 2% of its value, most preferably the term "about" means exactly the indicated value (± 0%).
The following examples serve to illustrate the present invention and should not be construed as limiting the scope thereof.

### EXAMPLES

### Example 1 - CovidGel test development

A multidisciplinary cooperation was established for the creation of a hydro-alcoholic hand sanitizing gel that meets current requirements in terms of its composition (25). Based on the literature and habits of our Mediterranean study population it was determined that the most suitable odoriferous substance was lemon (26).

### Material and Methods

### Hydrogel composition

- Distilled water: 24,2%
- Carbopol 0,5%
- Ethyl alcohol 70,0%
- Propylene glycol 5,0%
- Essential oil (Limón España Aceite Esencial 00281-6. LUCTA) 0,3%
- Triethanolamine c.s. to pH 6,5-7,5

Distilled water was added slowly and with continuous stirring through a glass rod to Carbopol^{®} until a transparent and viscous solution was obtained. Subsequently were added the ethyl alcohol, propylene glycol, triethanolamine and the essential oil, observing an increase in the viscosity of the mixture. Water percentage is modified accordingly in those formulations in the absence of Carbopol or using a different percentage of essential oil.

### Results

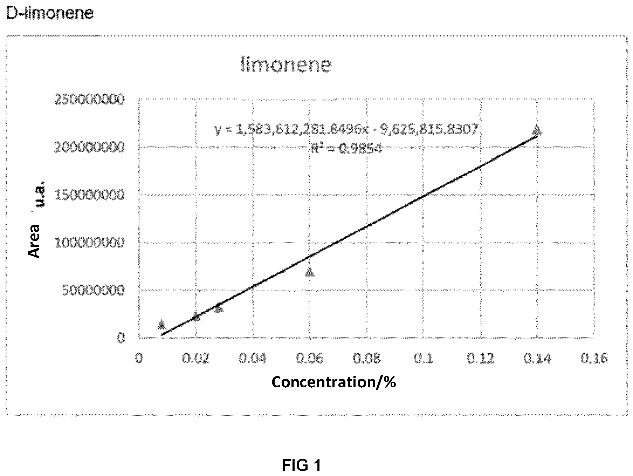

The selected lemon essential oil, limón España (Limón España Aceite Esencial 00281-6. LUCTA), comprises the following 4 volatile components: α-pinene, β-pinene, D-limonene and α-terpinene. D-limonene is the odoriferous volatile compound in the composition. Gas chromatography and mass spectrometry (GC-MS) were used to obtain semi-quantitative results, using a headspace sampling technique, to establish the effectiveness of the volatile odoriferous substance to evaporate from the hydroalcoholic gel at 37 C. As shown in Figure 1, it was found that D-limonene's concentration in the volatile fraction increased in a lineal manner with the concentration of the citric essential oil in the hydrogel.

Moreover, tests were carried out with different concentrations of lemon essential oil and lemon fragrances of chemical origin. The composition of the gel was adapted to attenuate the smell of alcohol. A GC-MS quantitative analysis was further conducted to test whether addition of a thickener had any impact on the volatility of D-limonene.

As shown in Table 1 below, the use of a thickening agent (i.e., Carbopol^{®} polymer) did not alter in a significant manner the concentration of D-limonene in the volatile fraction. From a hydroalcoholic gel at 0.3% it was detected 0.026% D-limonene in the gas phase and for the one at 0.5%, 0.047% of D-limonene.

**Table 1. - Percentage of the 4 main components' in the volatile fraction of the 0.3% and 0.5% essential oil compositions with and without thickener.**

| **Sample** | **component** | **calculated concentration %** |
|---|---|---|
| HYDROALCOHOLIC GEL 0.3% LEMON ESSENTIAL OIL | α-pinene | 0.0266 |
| | β-pinene | 0.0282 |
| | D-limonene | 0.0260 |
| | α-terpinene | 0.0250 |
| HYDROALCOHOLIC GEL 0.5% LEMON ESSENTIAL OIL | α-pinene | 0.0482 |
| | β-pinene | 0.0514 |
| | D-limonene | 0.0470 |
| | α-terpinene | 0.0426 |
| HYDROALCOHOLIC GEL + CARBOPOL^{®} 0.3% LEMON ESSENTIAL OIL | α-pinene | 0.0243 |
| | β-pinene | 0.0269 |
| | D-limonene | 0.0244 |
| | α-terpinene | 0.0217 |
| HYDROALCOHOLIC GEL + CARBOPOL^{®} 0.5% LEMON ESSENTIAL OIL | α-pinene | 0.0418 |
| | β-pinene | 0.0472 |
| | D-limonene | 0.0434 |
| | α-terpinene | 0.0385 |

On the basis of the obtained results, the hydroalcoholic gel with thickener was selected for the COVID-19 prospective study. In particular, two hydroalcoholic gels with increasing concentrations of lemon essential oil (0.3% and 0.5% of limón España (Limón España Aceite Esencial 00281-6. LUCTA), respectively) were used as olfactory test.

### Example 2 - Development and internal validation of a predictive model for COVID-19 population screening based on symptoms and a simple olfactory test

### Material and Methods

### Study design and setting

This is a population based prospective cohort study conducted following transparent TRIPOD Statement for transparent reporting of multivariable prediction models (23). The study was carried out in the Emergency Department of Sant Joan University Hospital of Reus, which is the reference hospital of the region and in all the 5 primary care centres of the public health sanitary net distributed in 5 basic health areas of the city of Reus.

The municipality of Reus is located in the Mediterranean area, has a surface area of 52.82 km² and at the beginning of 2020 it had a population of 106.168 inhabitants and a density of 2010.0 inhabitants/km² (24). This study was approved by the Ethics Committee of the Pere i Virgili Health Research Institute (Ref: 120/2020) and the IDIAP Jordi Gol Clinical Research Ethics Committee (Codi: 20/114-PCV). Funding for this study was not received. An informed consent signed was required for all the participants in the study.

### Participants

Consecutive patients undergoing RT-PCR for the first time to rule out COVID-19 infection who consulted the hospital emergency department or their primary care centre between June 15 and September 11, 2020 were included. Patients were tested for presenting symptoms suggestive of COVID-19 or for being close contacts of a confirmed COVID-19 case. Close contacts were considered those persons who had shared space with a positive case at a distance of less than 2 meters, for more than 15 minutes, without protection and from 48 hours prior to the onset of symptoms.

Patients excluded of the study protocol were those under 18 years of age and patients with pathologies or conditions that may interfere with olfactory function such as any degree of cognitive impairment, Parkinson's disease, chronic rhinosinusopathy, head trauma, nasal obstruction, treatment with high concentrations of oxygen, acute respiratory failure, altered state of consciousness, use of inhaled corticosteroids or those ones who did not sign the informed consent form.

### Characteristics of study population

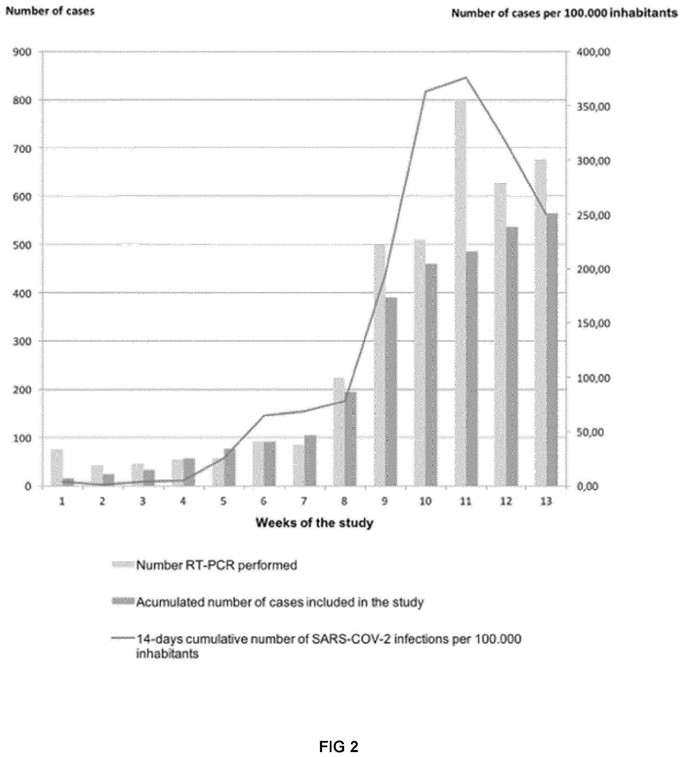

During the study period 3788 patients underwent RT-PCR to diagnose COVID-19 at one of the study health centres. The inclusion of cases and RT-PCRs performed per week in the centres while participating in the study is shown in **Figure 2****.**

The mean (SD) age of the study population was 42.3 (16.3) years, the range age was between 18 and 98 years and 48% were male. Table 2 shows the background and clinical characteristics of the study population. A greater percentage of males among the COVID-19 positive was found in respect to the COVID-19 negative patients (58.1% vs 45%) with an absolute difference of 13.09% (95% Cl 2.92 to 23.27, p=0.02).

Regarding medical background, a higher percentage of active smokers was found among COVID-19 negative patients (9.6% vs. 18%) with an absolute difference of 8.41% (95% Cl - 14.98 to -1.83; P=0.04) and a higher percentage of patients treated with chronic corticotherapy was found among COVID-19 positive patients (9.6% vs. 1.3%) with an absolute difference of 8.3% (95% Cl 2.81 to 13.79; P<0.01).

The majority of the patients presented a mild disease severity, summing up 334 cases (64.5%). A higher percentage of moderate severity cases was found among COVID-19 positive patients (18.8 % vs. 2%). No patients died during hospital admission. The most frequent diagnosis in the total study population was upper respiratory tract infection (12.7%). Pneumonia was diagnosed in 18% of the COVID-19 positive patients and in 1.7% of the COVID-19 negative patients with an absolute difference of 16.2% (95% Cl 9.1 to 23.2).

**Table 2. Background and Clinical Characteristics of the Study Population**

| | **Total** | **SARS-CoV2 positive** | **SARS-CoV2 negative** | |
|---|---|---|---|---|
| **Variable** | **N (%)** | **N=117** | **N=402** | **Absolute difference (95% CI). %** |
| **Demographic data** | | | | |
| Male patients | 249 (48) | 68 (58.1) | 181 (45) | 13.09 (2.92 to 23.27) |
| Age. Years | 42.3 (16.3) | 43.4 (15.95) | 41.9 (16.3) | 1.51 (-1.82 to 4.83) |

| **Background** | | | | |
|---|---|---|---|---|
| Hypertension | 96 (18.8) | 24 (20.9) | 72 (18.2) | 2.64 (-5.7 to 10.99) |
| Diabetes | 39 (7.6) | 9 (7.8) | 30 (7.6) | 0.23 (-5.33 to 5.79) |
| Dyslipidemia | 57 (11.2) | 16 (13.9) | 41 (10.4) | 3.53 (-3.47 to 10.54) |
| Smoking | 82 (16.1) | 11 (9.6) | 71 (18) | -8.41 (-14.98 to -1.83) |
| Enolism | 18 (3.5) | 8 (7) | 10 (2.5) | 4.42 (-0.48 to 9.33) |
| Chronic bronchopathy | 60 (11.8) | 13 (11.3) | 47 (11.9) | -0.59 (-7.2 to 6.02) |
| Chronic heart disease | 28 (5.5) | 5 (4.3) | 23 (5.8) | -1.47 (-5.86 to 2.91) |
| Neoplasia | 19 (3.7) | 4 (3.5) | 15 (3.8) | -0.32 (-4.16 to 3.52) |
| Autoinmune disease | 14 (2.7) | 6 (5.2) | 8 (2) | 3.19 (-1.1 to 7.49) |
| Chronic renal failure | 4 (0.8) | 0 (0) | 4 (1) | -1.01 (-2 to -0.03) |
| Chronic liver disease | 14 (2.7) | 5 (4.3) | 9 (2.3) | 2.07 (-1.94 to 6.08) |
| Hypothyroidism | 26 (5.1) | 3 (2.6) | 23 (5.8) | -3.21 (-6.93 to 0.5) |
| Obesity | 43 (8.3) | 11 (9.4) | 32 (8) | 1.44 (-4.47 to 7.35) |
| Chronic cortico-therapy | 16 (3.1) | 11 (9.6) | 5 (1.3) | 8.3 (2.81 to 13.79) |
| Immunosuppressive therapy | 5 (0.98) | 2 (1.7) | 3 (0.8) | 0.96 (-1.55 to 3.48) |

| **Disease severity** | | | | |
|---|---|---|---|---|
| Mild | 334 (64.5) | 74 (63.2) | 260 (64.8) | -1.59 (-11.5 to 8.32) |
| Moderate | 30 (5.8) | 22 (18.8) | 8 (2) | 16.81 (9.6 to 24.02) |
| Severe | 6 (1.2) | 2 (1.7) | 4 (1) | 0.71 (-1.83 to 3.25) |
| Oxygen therapy | 38 (7.34) | 21 (17.9) | 17 (4.2) | 13.71 (6.48 to 20.94) |

| **Diagnosis** | | | | |
|---|---|---|---|---|
| Upper respiratory tract infection | 66 (12.7) | 34 (29.1) | 32 (8.0) | 21.1 (12.5 to 29.7) |
| Lower respiratory tract infection | 22 (4.2) | 2 (1.7) | 20 (5.0) | -3.3 (-6.4 to -0.10) |
| Pneumonia | 28 (5.4) | 21 (18.0) | 7 (1.7) | 16.2 (31.5 to 50.5) |

| | | | | |
|---|---|---|---|---|
| Values are median (Standart Deviation) and n (%). | | | | |

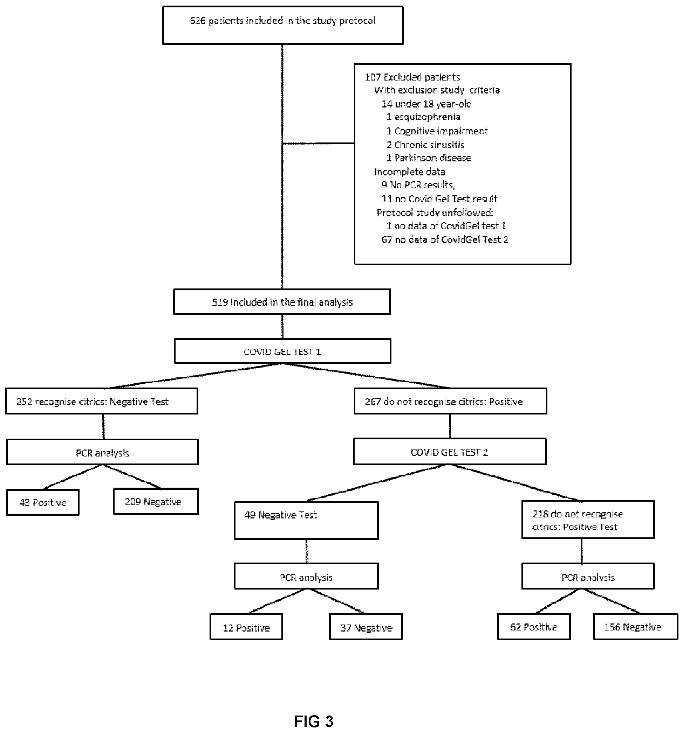

**Figure 3** provides a Flow Chart summarizing the results of the study. 626 patients were initially included in the study protocol. A total of 107 patients were excluded because of incomplete data or exclusion criteria. The final analysis of the study included 519 patients out of whom 341 patients (65.7%) were from the primary care and 179 (34.3%) from the hospital Emergency Department. Regarding the reasons for conducting a RT-PCR test, 386 (74.4%) had at least one symptom suggestive of COVID-19, 118 (22.7%) were asymptomatic and were close contacts of a COVID-19 case and 15 (2.9%) were asymptomatic and were tested for unknown reasons. A positive RT-PCR was found in 117 patients (22.5%) and a negative RT-PCR was found in 402 patients (77.5%).

### Description of the olfactory test

The olfactory test was performed by properly trained primary care and emergency nurses prior to the collection of the sample for SARS-COV-2 RT-PCR. Therefore, both the patient and the healthcare personnel were blinded to the patient infection status. Firstly, the test consisted on the application of 1 ml of 0.3% CovidGel using a dispenser on the patient hands palm. After hand massaging and a 3-second-pause, the patient was asked to smell their hands. Subsequently the following instruction was made: *'Please, identify the smell of this gel'.* The answer is recorded on the basic data collection sheet regardless of the result. If the answer was not lemon or if it was inconclusive, the same test was repeated after 30 seconds with the 0.5% CovidGel. The olfactory test was considered negative if the patient recognized a citrus fruit, and the olfactory test was considered positive if the patient failed and did not recognize a citrus fruit.

### Data collection

A data collection sheet was completed by the attending nurse before taking the sample for the RT-PCR Test. It included the results of the two olfactory tests when both were performed. It also included age, gender, duration of symptoms (in days), and a yes or no questionnaire to check for symptoms such as fever, dry cough, dyspnea, anorexia, myalgia, headache, diarrhea, asthenia, productive cough, sore throat, OD or gustatory dysfunction (GD), others or non-symptoms. The RT-PCR test for the detection of SARS-COV-2 was considered the gold standard for diagnosis. During our study, the RT-PCR was performed by trained personnel based on the technical considerations of the manufacturer using a double sampling of the pharynx and the nose. The conservation of the sample and the transfer to the laboratory followed the channels of the usual clinical practice from the center. RT-PCR tests were carried out with the VIASURE SARS-COV-2 Real Time PCR Detection Kit (CerTest Biotec, Zaragoza, Spain), or with the Procleix1 method in a Panther automated extractor and amplifier (Grifols Laboratories, Barcelona, Spain). Once all the data collection sheets were finished, the medical digital records were consulted allowing to register the RT-PCR test results, backgrounds, evolution of the patients and discharge diagnosis. Regarding the severity of the disease, the patients attended and discharged immediately are considered mild, those admitted to the hospital as moderate and those requiring ICU during hospitalization as severe. This study was conducted at the beginning of the second wave of COVID-19 in our region (27). The 14-day cumulative incidence of COVID-19 cases in the city of Reus increased gradually from 0.9 cases/100.000 inhabitants in June 15 to 376.09 cases/100.000 inhabitants in August 24 (28).

### Statistical analysis

Quantitative variables used in this study were described using the mean, the standard deviation, the median and the first and third quartiles. The difference between means and its corresponding 95% confidence interval (CI) were also used to compare groups of patients. Categorical variables were described using the number of cases, percentages and 95% Cl. Comparisons between groups of patients were performed using Student's T test for quantitative variables, while the chi-squared test was used for categorical variables. Groups of patients were also compared in terms of risk difference and odds ratio of binary variables, and their corresponding 95% Cl. All tests were two-tailed and p-values lower than 0.05 were considered statistically significant.

Diagnostic values in terms of sensitivity, specificity, positive predictive value, negative predictive value, positive likelihood ratio and negative likelihood ratio, as well as their corresponding 95% Cl, were calculated for binary variables and smell tests.

Symptoms that proved to be statistically significant in a logistic regression predictive model, were fever, dry cough, myalgia, headache, diarrhoea, asthenia, perceived altered sense of smell, and perceived altered sense of taste and were defined as relevant. The productive cough variable was also included as a relevant symptom. The number of relevant symptoms was counted for each patient and this new variable was used to develop the model based on classification trees using a recursive partitioning algorithm (29). The internal model validation was made using cross validation techniques in machine learning avoiding overfitting over-adjustment during the construction of the classification trees. These classification trees were built using the following parameters: the splitting index was Gini coefficient; the minimum number of patients in any node of a tree for a split to be attempted was set to 30; the minimum number of patients in any terminal node of a tree was set to 10; node splits were only attempted if they improved the fit by a factor of 0.01; and the number of cross-validations to be run was set to 10.

In order to obtain different values of sensitivity and specificity in the resulting classification trees, distinct costs of false positives and false negatives were used in the loss matrix parameter of the classification tree algorithm. In particular, for the high sensitive classification tree the cost for false negatives was set in 1:8, while the cost for false positives was kept constant to 1.

To handle missing data in the study protocol, several predictive models were analyzed, adopting a data-complete analysis over other strategies due to the low relevance of the missing data in the final results of the predictive machine learning model. All statistical analyses were performed using R software version 4.0.

### Results

### Association of symptoms with COVID-19

The mean (SD) days of the symptom's evolution was 5.8 (5.6) for the SARS-COV2 positive patients and 5.1 (12.1) for the SARS-COV2 negative patients with an absolute difference of 0.75 (95% Cl -1.35 to 2.84; P=0.48). The symptoms most strongly associated with COVID-19 were perceived olfactory disfunction (OD) and perceived gustatory disfunction (GD) (OR 5.78; 95% Cl 2.76- 12.12, P<0.01 and OR 5.78; 95% Cl 3.03-11.04, P<0.01, respectively) followed by fever (OR 3.03; 95% Cl 1.98-4.65, P<0.01). Fever, dry cough, asthenia, myalgia, headache, diarrhea, OD, and GD were the 8 symptoms found to be associated with COVID-19. Table 3 shows the reported symptoms and olfactory test results in the population study.

**Table 3. Patient-Reported of COVID-19 Symptoms and Olfactory Test Results**

| | **SARS-COV2 positive** | **SARS-COV2 negative** | | |
|---|---|---|---|---|
| | N=117 | N=402 | **Odds Ratio (95% CI)** | **p-value** |
| **Symptoms** | | | | |
| **Fever** | 59 (50.4) | 101 (25.1) | 3.03 (1.98-4.65) | 0.00 |
| **Dry cough** | 45 (38.5) | 73 (18.2) | 2.82 (1.8-4.42) | 0.00 |
| **Asthenia** | 34 (29.1) | 60 (14.9) | 2.33 (1.44-3.79) | 0.00 |
| **Myalgias** | 30 (25.6) | 61 (15.2) | 1.93 (1.17-3.17) | 0.01 |
| **Cephalea** | 39 (33.3) | 83 (20.6) | 1.92 (1.22-3.03) | 0.01 |
| **Diarrhea** | 35 (29.9) | 82 (20.4) | 1.67 (1.05-2.65) | 0.04 |
| **OD** | 19 (16.2) | 13 (3.2) | 5.79 (2.76-12.12) | 0.00 |
| **GD** | 25 (21.4) | 18 (4.5) | 5.78 (3.03-11.04) | 0.00 |
| Dyspnea | 23 (19.7) | 54 (13.4) | 1.58 (0.92-2.7) | 0.13 |
| Productive cough | 15 (12.8) | 40 (10) | 1.33 (0.7-2.5) | 0.48 |
| Sore throat | 31 (26.5) | 96 (23.9) | 1.15 (0.72-1.84) | 0.65 |
| Rhinorrhoea | 6 (5.1) | 9 (2.2) | 2.36 (0.82-6.77) | 0.18 |
| Anorexia | 10 (8.5) | 30 (7.5) | 1.16 (0.55-2.45) | 0.85 |
| Asymptomatic | 13 (11.1) | 120 (29.9) | 0.29 (0.16-0.54) | 0.00 |

| **Symptoms combination** | | | | |
|---|---|---|---|---|
| GD and OD | 31 (26.5) | 24 (6) | 5.66 (3.16-10.13) | 0.00 |
| Fever and dry cough | 75 (64.1) | 146 (36.3) | 3.13 (2.04-4.81) | 0.00 |
| Fever. dry cough and OD | 82 (70.1) | 152 (37.9) | 3.84 (2.46-5.98) | 0.00 |

| **Olfactory Test results** | | | | |
|---|---|---|---|---|
| Test 1 positive | 74 (63.2) | 193 (48) | 1.86 (1.22-2.85) | 0.01 |
| No smell at all | 13 (11.1) | 12 (3) | 4.06 (1.8-9.17) | 0.00 |
| Test 1 and 2 positive | 62 (52.9) | 156 (38.8) | 1.78 (1.17-2.69) | 0.01 |

| | | | | |
|---|---|---|---|---|
| OD Olfactory disfunction; GD Gustatory disfunction; Values are n (%) | | | | |

### Olfactory test results and diagnostic values of symptoms and olfactory test

In the total population study, the *CovidGel test 1* was positive in 267 patients (51.4%) and negative in 252 patients (48.6%). Among patients with a positive olfactory test result 112 cases (41.9%) identified the gel smell as alcohol, 57 cases (21.3%) as cologne, 27 cases (10.1%) as aromatic herbs, 10 cases (3.7%) as non-citrus fruits (3.7%), 6 cases (2.2%) as alcoholic beverages (2.2%) and 22 cases (8.2%) as other responses. In 25 cases (9.4%) "don't smell at all" were reported and in 8 cases a "don't know" response (3%) was reported. Among patients with a negative olfactory test result, 207 cases (82.1%) identified the gel smell as lemon, 26 cases (10.3%) as citrus, 13 cases (5.1%) as orange, 2 cases (0.8%) as tangerine, 2 cases (0.8%) as citronella, and 2 cases (0.8%) as lime.

Among the 117 patients diagnosed with COVID-19, the *CovidGel Test 1* was positive in 74 (63.2%) and among the 402 non-COVID-19 patients, *CovidGel Test 1* was positive in 193 patients (48%). Positive CovidGel test 1 was associated with COVID-19 (OR: 1.86; 95% Cl 1.22-2.85, P<0.01). A positive CovidGel Test 1 and 2 resulted in a lower association with COVID-19 (OR: 1.78; 95% Cl 1.17-2.69, P<0.01). The response "not smell at all" was strongly associated with COVID-19 (OR: 4.06; 95% Cl 1.8 - 9.17).

Table 4 shows the olfactory test results in relation to symptoms. Among the 13 asymptomatic COVID-19 positive patients, 10 (76.9%) had a positive *CovidGel Test* result. CovidGel Test 1 positive results or both CovidGel Test 1 and 2 positive results in asymptomatic patients were associated with COVID-19 (OR: 3.94; 95% Cl 1.03-15.03 and OR: 6.19; 95% Cl 1.62-23.73 respectively) and Table 5 shows the diagnostic values of the relevant symptoms, the combination of symptoms and olfactory test to predict COVID-19.

**Table 4. Olfactory test in relation to symptoms**

| | SARS-COV2 positive | SARS-COV2 negative | Odds ratio (95% CI) |
|---|---|---|---|
| **All patients** | N=117 | N=402 | |
| Test 1 positive | 74 (63.2) | 193 (48) | 1.86 (1.22-2.85) |
| No smell at all | 13 (11.1) | 12(3) | 4.06 (1.8-9.17) |
| Test 1 and 2 positive | 62 (52.9) | 156 (38.8) | 1.78 (1.17-2.69) |

| **Symptomatic patients** | N=104 | N=282 | |
|---|---|---|---|
| Test 1 positive | 64 (61.54) | 138 (48.94) | 1.67 (1.06-2.64) |
| No smell at all | 12 (11.5) | 12 (4.3) | 2.93 (1.27-6.76) |
| Test 1 and test 2 positive | 50 (50.0) | 114 (40.4) | 1.47 (0.94-2.32) |

| **Asymptomatic patients** | N= 13 | N= 120 | |
|---|---|---|---|
| Test 1 positive | 10 (76.9) | 55 (45.8) | 3.94 (1.03-15.03) |
| No smell at all | 1 (7.7) | 0 (0) | |
| Test 1 and 2 positive | 10 (76.9) | 42 (35) | 6.19 (1.62-23.73) |

**Table 5 Relevant Symptoms and Olfactory Test Diagnostic Values**

| **Symptoms** | **Sensitivity (95% CI)** | **Specificity (95%CI)** | **PPV (95% CI)** | **NPV (95% CI)** | **PLR** | **NLR** |
|---|---|---|---|---|---|---|
| Fever | 0.50 (0.41-0.6) | 0.75 (0.7-0.79) | 0.37 (0.29-0.45) | 0.84 (0.8-0.87) | 2.01 | 0.66 |
| Dry cough | 0.38 (0.3-0.48) | 0.82 (0.78-0.85) | 0.38 (0.29-0.48) | 0.82 (0.78-0.86) | 2.12 | 0.75 |
| Asthenia | 0.29 (0.21-0.38) | 0.85 (0.81-0.88) | 0.36 (0.27-0.47) | 0.8 (0.76-0.84) | 1.95 | 0.83 |
| Myalgias | 0.26 (0.18-0.35) | 0.85 (0.81-0.88) | 0.33 (0.23-0.44) | 0.8 (0.76-0.83) | 1.69 | 0.88 |
| Cephalea | 0.33 (0.25-0.43) | 0.79 (0.75-0.83) | 0.32 (0.24-0.41) | 0.8 (0.76-0.84) | 1.61 | 0.84 |
| Diarrhea | 0.3 (0.22-0.39) | 0.8 (0.75-0.83) | 0.3 (0.22-0.39) | 0.8 (0.75-0.83) | 1.47 | 0.88 |
| OD | 0.16 (0.1-0.24) | 0.97 (0.95-0.98) | 0.59 (0.41-0.76) | 0.8 (0.76-0.83) | 5.01 | 0.87 |
| GD | 0.21 (0.14-0.3) | 0.96 (0.93-0.97) | 0.58 (0.42-0.73) | 0.81 (0.77-0.84) | 4.76 | 0.82 |

| **Symptoms combination** | | | | | | |
|---|---|---|---|---|---|---|
| OD and GD | 0.26 (0.19-0.35) | 0.94 (0.91-0.96) | 0.56 (0.42-0.7) | 0.81 (0.78-0.85) | 4.43 | 0.78 |
| Fever and dry cough | 0.64 (0.55-0.73) | 0.64 (0.59-0.68) | 0.34 (0.28-0.41) | 0.86 (0.81-0.9) | 1.77 | 0.56 |
| Fever dry cough and OD | 0.7 (0.61-0.78) | 0.62 (0.57-0.67) | 0.35 (0.29-0.42) | 0.88 (0.83-0.91) | 1.85 | 0.48 |

| **Olfactory test results** | | | | | | |
|---|---|---|---|---|---|---|
| Test 1 positive | 0.63 (0.54-0.72) | 0.52 (0.47-0.57) | 0.28 (0.22-0.33) | 0.83 (0.78-0.87) | 1.32 | 0.71 |
| Test 1 and 2 positive | 0.53 (0.44-0.62) | 0.61 (0.56-0.66) | 0.28 (0.23-0.35) | 0.82 (0.77-0.86) | 1.37 | 0.77 |

### Results of the machine learning predictive model

Table 6 shows the results of the different classification trees built by machine learning according to the variables introduced in the model. By only introducing the 8 relevant symptoms into the model, the sensitivity was 0.86 (95 Cl 0.79-0.92), the specificity was 0.37 (95% Cl 0.33-0.42) and the AUC was 0.86 (0.81-0.9) for the total population study and 0.97 (95%CI 0.92-0.99), 0.11 (95% Cl 0.07-0.15) and 0.89 (95% Cl 0.84-0.93) respectively for the symptomatic population. Sensitivity raised to 0.94 (95%CI 0.88-0.98) whilst specificity remained at 0.32 (95% Cl 0.28-0.37) when the olfactory test was introduced into the model for the total population study. The sensitive classification tree built only took into account the result of CovidGel Test 1 and ignored the result of CovidGel Test 2. Considering other clinical variables, the model also included sex and age, reaching a sensitivity of 0.97 (0.91-0.99), a specificity of 0.39 (0.34-0.44) and an AUC of 0.87 (95% Cl 0.83-0.92) for the total population study and 0.98 (95%CI 0.93-1), 0.31 (95% Cl 0.26-0.37) and 0.89 (95% Cl 0.84-0.93) for the symptomatic population. respectively.

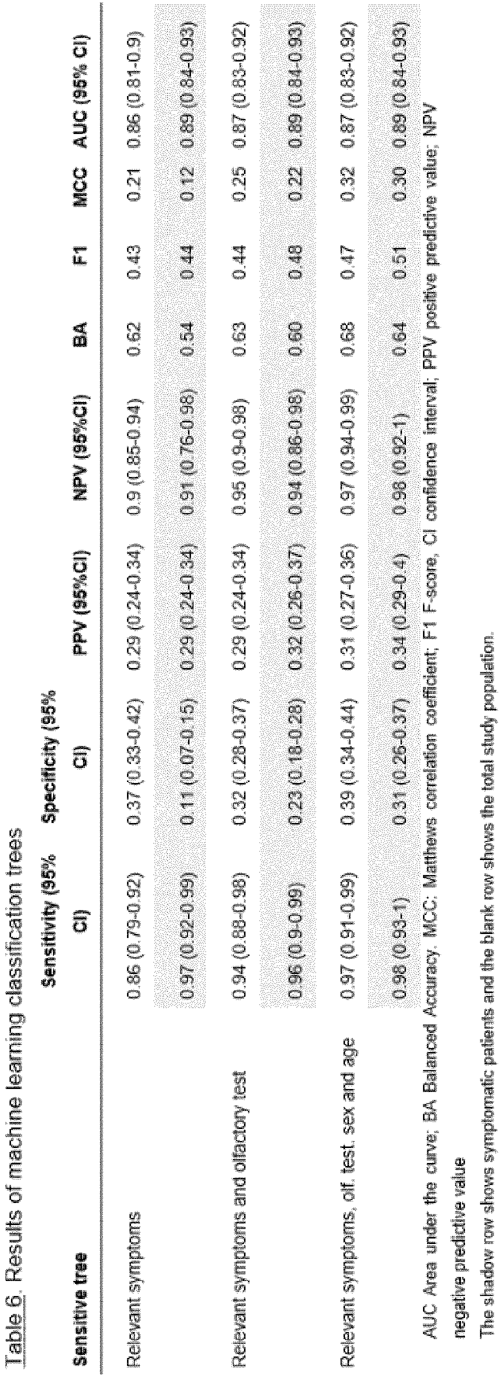

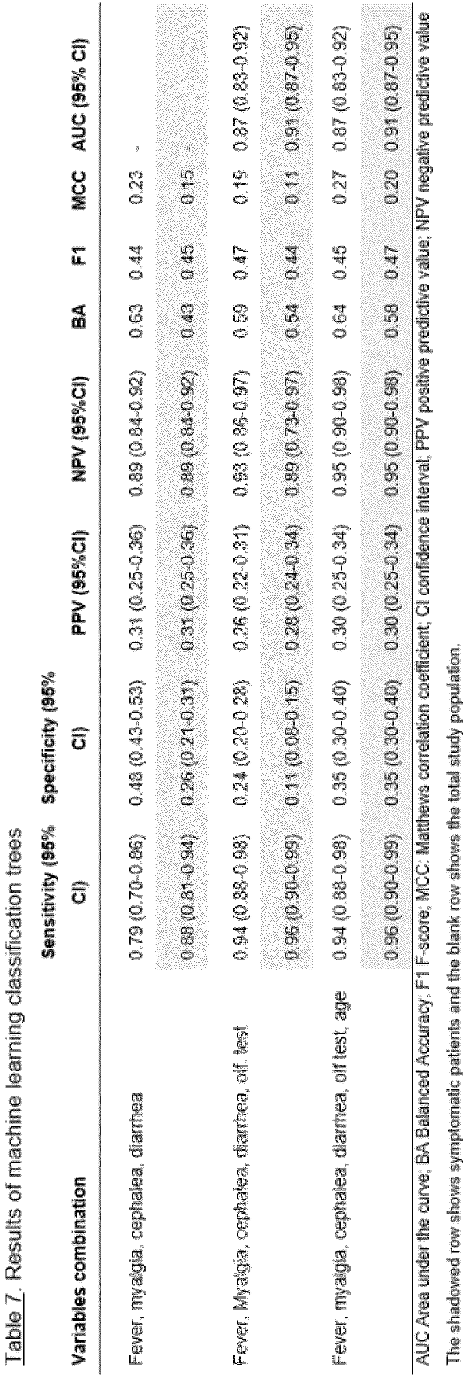

Table 7 shows the results of additional sensitivity classification trees built by machine learning according to the variables introduced in the model. By only introducing the 4 relevant symptoms into the model, the sensitivity was 0.79 (95 Cl 0.70-0.86) and the specificity was 0.48 (95% Cl 0.43-0.53) for the total population study. There was a striking raise in sensitivity to 0.94 (95%CI 0.88-0.98) when the olfactory test was introduced into the model for the total population study although specificity decreased to 0.24 (95% Cl 0.20-0.28). Again, the sensitive classification tree built only took into account the result of CovidGel Test 1 and ignored the result of CovidGel Test 2. Considering other clinical variables, the model also included age, which enabled increasing specificity to 0.35 (0.30-0.40) for the total population study.

### Discussion

The combination of symptoms and a simple olfactory test based on the identification of the smell of a hydroalcoholic gel has allowed the development of a predictive model with high sensitivity, which has important clinical implications.

A predictive model based on symptoms reported in a smartphone-based app obtained a sensitivity of 0.65 (95% Cl 0.62-0.67) and an AUC of 0.76 (95% Cl 0.74-0.78) to predict COVID-19 (14). Another predictive model using machine learning based on symptoms, gender, age and close contacts obtained a sensitivity between 0.85 and 0.87 depending on the possible working points and an AUC of 0.86 (95% Cl 0.85-0.87) (10). An advantage of our predictive model is that it does not use close contacts as a variable. It should be considered that since COVID-19 can be asymptomatic, oftentimes the patient is not diagnosed, and the tested subject is therefore not aware of having been in close contact with a SARS-COV-2 positive individual.

The different results of our model depending on the variables included present similar or even higher diagnostic values with respect to these models proposed as population screening. To our knowledge, this is the first model that includes an olfactory test built using a prospective population-based study.

Without willing to be bound by theory, the high sensitivity in our model was obtained thanks to the low false negative rate of the olfactory test among asymptomatic COVID-19 positive patients. The CovidGel Test obtained a sensitivity almost twice as high in comparison with a more complex olfactory test to predict COVID-19 based on the identification of the smell of 3 scented paper strips and a 4-item scale intensity rate (15). In addition, the simplicity of the CovidGel test enables its implementation as a self-test, making it a more suitable population screening olfactory test than any reported before. A wide distribution of the CovidGel due to is low cost can also contribute to improving the disease situational awareness of the population.

It is important to highlight that in our study no side effects related to the inhalation of the hydroalcoholic gel were reported. One study described that repeated exposure to a hydroalcoholic gel by inhalation does not increase blood ethanol levels (20). The side effects described in the literature are related to the occurrence of dermatitis or are due to their ingestion (21) (22).

### Bibliograpic References

1. Zhu, N., et al. A Novel Coronavirus from Patients with Pneumonia in China, 2019. N Engl J Med. 382, 727-733 (2020).
2. World Health Organization. COVID-19 Weekly Epidemiological Update https://www.who.int/publications/m/item/weekly-epidemiological-update-on-covid-19---18-may-2021(2021).
3. Rubin, E.J., Baden, L.R. & Morrissey, S. Audio Interview: Covid-19 Vaccine Fundamentals. N Engl J Medicine. 383, e146 (2020).
4. Speiser, D.E., & Bachmann, M.F. COVID-19: Mechanisms of Vaccination and Immunity. Vaccines. 8,404 (2020).
5. Lauring, A.S. & Hodcroft, E.B. Genetic Variants of SARS-CoV-2-What Do They Mean?, JAMA. 325,529-531(2021).
6. The Lancet. India's COVID-19 emergency. The Lancet. 397, 1683 (2021).
7. Rapid and frequent testing. Nat Biomed Eng. 4, 1121-1122 (2020).
8. Gandhi, M., Yokoe, D.S. & Havlir, D.V. Asymptomatic Transmission, the Achilles' Heel of Current Strategies to Control Covid-19. N Engl J Med. 382, 2158-2160 (2020).
9. Pollán, M., et al. Prevalence of SARS-CoV-2 in Spain (ENE-COVID): a nationwide, population-based seroepidemiological study. The Lancet. 396, 535-544 (2020).
10. Zoabi, Y., Deri-Rozov, S., & Shomron, N. Machine learning-based prediction of COVID-19 diagnosis based on symptoms. npj Digit Med. 4, 3 (2021).
11. Quer, G,, et al. Wearable sensor data and self-reported symptoms for COVID-19 detection. Nat Med. 27, 73-77 (2021).
12. Lechien, J.R., et al. Olfactory and gustatory dysfunctions as a clinical presentation of mild-to-moderate forms of the coronavirus disease (COVID-19): a multicenter European study. Eur Arch of Otorhino-Laryngology. 277, 2251-2261(2020)
13. Spinato, G., et al. Alterations in Smell or Taste in Mildly Symptomatic Outpatients With SARS-CoV-2 Infection. JAMA. 323, 2089 (2020).
14. Menni, C., et al. Real-time tracking of self-reported symptoms to predict potential COVID-19. Nature Medicine. 26, 1037-1040 (2020).
15. Villerabel, C., et al. Diagnostic Value of Patient-Reported and Clinically Tested Olfactory Dysfunction in a Population Screened for COVID-19. JAMA Otolaryngol Head Neck Surg. 147:271-279 (2021).
16. Kaye, R., et al. COVID-19 Anosmia Reporting Tool: Initial Findings. Otolaryngology-Head and Neck Surg. 163, 132-134 (2020).
17. Golin, A.P., Choi, D. & Ghahary, A. Hand sanitizers: A review of ingredients, mechanisms of action, modes of delivery, and efficacy against coronaviruses. American Journal of Infection Control. 48, 1062-1067 (2020).
18. Astani, A,, Reichling, J. & Schnitzler. P. Comparative study on the antiviral activity of selected monoterpenes derived from essential oils: Antiviral activity of monoterpenes derived from essential oils. Phytotherapy Research. 24, 673-679 (2010).
19. da Silva JKR, Figueiredo PLB, Byler KG, & Setzer WN. Essential Oils as Antiviral Agents, Potential of Essential Oils to Treat SARS-CoV-2 Infection: An In-Silico Investigation. International Journal of Molecular Sciences. 21, 3426 (2020).
20. Huynh-Delerme C, et al. Short Communication: Is Ethanol-Based Hand Sanitizer Involved in Acute Pancreatitis after Excessive Disinfection?-An Evaluation with the Use of PBPK Model. J Toxicol. 2012, 1-7 (2012);2012:1-7.
21. Emadi A & Coberly L. Intoxication of a Hospitalized Patient with an Isopropanol-Based Hand Sanitizer. N Engl J Med. 356, 530-531.
22. Quenan S & Piletta P. Hand dermatitis in healthcare workers: 15-years experience with hand sanitizer solutions. Contact Dermatitis. 84, 339-340 (2020).
23. Collins GS, Reitsma JB, Altman DG, & Moons K. Transparent reporting of a multivariable prediction model for individual prognosis or diagnosis (TRIPOD): the TRIPOD Statement. BMC Med. 13, 1 (2015).
24. IDESCAT. Statistical Institute of Catalonia. https://www.idescat.cat/emex/?id=431233&lang=en#h1f (2021)
25. European Chemicals Agency. Information on biocides https://echa.europa.eu/es/information-on-chemicals/biocidal-active-substances (2021)
26. Moein ST, et al.. Smell dysfunction: a biomarker for COVID-19. Int Forum of Allergy Rhinology. 10,944-950 (2020).
27. Iftimie S, et al. First and second waves of coronavirus disease-19: A comparative study in hospitalized patients in Reus, Spain. PLOS ONE.16, e0248029 (2021)
28. Gencat. Salut/Dades COVID. https://dadescovid.cat/setmanal?tipus_territori=territori&id_html=up_4_23&tipus=municipi&codi =43123 (2021)
29. Mola F. Classification and Regression Trees Software and New Developments. In: Rizzi A, Vichi M, Bock H-H, editors. Advances in Data Science and Classification [Internet]. Berlin, Heidelberg: Springer Berlin Heidelberg; 1998 [cited 2021 Apr 11]. p. 311-8. (Bock H-H, Gaul W, Opitz O, Schader M, editors. Studies in Classification, Data Analysis, and Knowledge Organization). http://link.springer.com/10.1007/978-3-642-72253-0_42
30. Dinnes J, Deeks JJ, Berhane S, Taylor M, Adriano A, Davenport C, Dittrich S, Emperador D, Takwoingi Y, Cunningham J, Beese S, Domen J, Dretzke J, Ferrante di Ruffano L, Harris IM, Price MJ, Taylor-Phillips S, Hooft L, Leeflang MM, Mclnnes MD, Spijker R, Van den Bruel A; Cochrane COVID-19 Diagnostic Test Accuracy Group. Rapid, point-of-care antigen and molecular-based tests for diagnosis of SARS-CoV-2 infection. Cochrane Database Syst Rev. 2021 Mar 24;3(3):CD013705.

## Claims

**1.** A computer implemented (CI) method for the screening of subjects infected with SARS-COV-2 virus, wherein said method comprises:
i. collecting information from a subject on the perceived presence of clinical symptoms, wherein said clinical symptoms comprise or consist of fever, myalgia, diarrhoea, headache, cough, asthenia, olfactory disfunction (OD), and gustatory disfunction (GD);
ii. collecting the results of an olfactory test requiring the identification of a scent by the subject, preferably wherein said olfactory test consists of a single testing step, wherein when the scent is not identified, the olfactory test is annotated as positive;
iii. processing the information obtained from steps i) and ii) by a computer to generate an output information identifying or classifying a subject having or with a high likelihood to have SARS-COV-2 infection;
preferably, wherein said subjects are from a random sample of the general population which includes symptomatic and asymptomatic individuals.

**2.** The method according to claim 1, wherein
when said subject has two or more of the clinical symptoms recited in step i); or
when said subject has zero or one of the clinical symptoms recited in step i) and has a positive olfactory test in ii);
an output information is generated in step iii) identifying said subject as having or with a high likelihood to have SARS-COV-2 infection.

**3.** The method according to any of claims 1 or 2, wherein
when said subject has in step i) fever, myalgia, diarrhoea and headache; and
has a positive olfactory test in ii);
an output information is generated in step iii) identifying said subject as having or with a high likelihood to have SARS-COV-2 infection.

**4.** The method according to any of claims 1 to 3, wherein step i) further comprises collecting information from the subject's age and/or sex; and wherein
when said subject has a negative olfactory test in ii) and it is a woman then said subject is identified as not having or without a high likelihood to have SARS-COV-2 infection; or when said subject has a negative olfactory test in ii) and it is a men then if he has more than 50 years old then it is identified as not having or without a high likelihood to have SARS-COV-2 infection and if he has 50 or less years is identified as having or with a high likelihood to have SARS-COV-2 infection.

**5.** The method according to any of claims 1 to 4, wherein sensitivity values are equal to or above 90%.

**6.** The method according to any of claims 1 to 5, wherein the subject identified or classified as having or with a high likelihood to have SARS-COV-2 infection is further submitted to a confirmatory diagnostic test, preferably to SARS-COV-2 RT- PCR.

**8.** The method according to any of claims 1 to 7, wherein the olfactory test requires the identification of a citric scent.

**9.** The method according to any of claims 7 or 8, wherein said scent arises from an aromatizing compound found in a concentration from 0.3% w/w or v/w to 0.5% w/w or v/w in said composition, preferably wherein the concentration of the aromatizing compound in the composition is about 0.3% w/w or v/w.

**10.** The method according to claim 9, wherein said aromatizing compound is an essential oil, preferably a citric essential oil, such as an orange, tangerine, lemon, citronella, or lime essential oil, more preferably wherein said essential oil is lemon essential oil.

**11.** A device for the screening of subjects infected with SARS-COV-2 virus, comprising:
one or more means for collecting the information regarding the clinical condition of the subject and the results of an olfactory test as described in any of claims 1 to 10, and
means for generating information to generate and output information,
wherein the means for generating information is constituted so that
A) during a first collection step, information relating to clinical conditions of the subject is collected, comprising the perceived presence of clinical symptoms, wherein said clinical symptoms comprise or consist of fever, myalgia, diarrhoea, headache, cough, asthenia, olfactory disfunction (OD), and gustatory disfunction (GD);
B) during a second collection step the results of an olfactory test requiring the identification of a scent by the subject are collected, wherein when the scent is not identified, the olfactory test is annotated as positive; and
C) processing the information collected in A) and B) and generating an output information identifying said subject as having or with a high likelihood to have SARS-COV-2 infection.

**12.** The device of claim 11, wherein
when said subject has two or more of the clinical symptoms recited in A); or
when said subject has zero or one of the clinical symptoms recited in A) and has a positive olfactory test in B);
an output information is generated in C) identifying said subject as having or with a high likelihood to have SARS-COV-2 infection.

**13.** The device of any of claims 11 or 12, wherein in step B) said olfactory test comprises a single testing step.

**14.** The device according to any of claims 11 to 13, wherein said means for collecting the information regarding the clinical condition is an online clinical questionnaire accessible to from a terminal device.

**15.** A computer program capable of implementing step iii) of the methods according to any of claims 1 to 10 or for generating the output information in C) of the device according to any of claims 11 to 14.
